# EUROPEAN PATENT SPECIFICATION

(11) **EP 1 713 575 B1**
(45) Date of publication and mention of the grant of the patent: **30.07.2008**
(21) Application number: 05700628.0
(22) Date of filing: 04.02.2005
(51) Int. Cl.: B01J 13/04

(54) **AQUEOUS DISPERSION AND ITS USE**
WÄSSRIGE DISPERSION UND IHRE VERWENDUNG
DISPERSION AQUEUSE ET SON UTILISATION

(30) Priority: 06.02.2004 DK 200400184; 06.02.2004 US 541947 P
(43) Date of publication of application: 25.10.2006
(73) Proprietor: BASF SE, 67056 Ludwigshafen (DE)
(72) Inventor: HANSEN, Carsten, Lynggaard, DK-2765 Smorum (DK)
(74) Representative: Wetke, Ellen
(86) International application number: PCT/DK2005/000078
(87) International publication number: WO 2005/075066

(56) References cited:
- EP-A- 0 922 449
- US-A- 4 035 235
- US-A- 5 720 978

## Description

### FIELD OF INVENTION

The present invention relates to an aqueous dispersion comprising an active substance dispersed in modified starch, a process of preparing such a dispersion as well as its use, e.g. for preparing microcapsules.

### BACKGROUND OF THE INVENTION

Naturally occurring and modified polysaccharides and naturally occurring hydrocolloids such as alginate, caseinate, carrageenan, gelatin, pectins, gum arabic, acacia gum, tragant, starch and modified starch, find wide spread use as matrix materials for use in the microencapsulation of sensitive or labile active substances such as vitamins, aroma and flavour substances in food, food supplements, pharmaceuticals and agricultural products, in order to protect them from exposure to oxygen, moisture and irradiation as well as physical influences, such as pressure, and thus to avoid chemical and/or physical degradation of said active substances and to improve their storage stability.

Microencapsulated products based on the use of matrix materials of plant origin are normally preferred for use in some products such as vegetarian foods, kosher foods and halal foods. They may also be advantageous to use as matrix materials instead of matrix materials of animal origin due to legislative restrictions.

One alternative to a matrix material of animal origin, described in the literature, is starch and modified starch such as depolymerised starch and starch hydrolysates, which may be used for dispersion purposes. A process for preparing dispersed products comprising starch and modified starch are also described in literature.

EP 1 066 761 A2 describes a composition comprising a fat soluble substance encapsulated in a carbohydrate matrix comprising maltose or a mixture of low-molecular weight carbohydrates, as well as a process for preparing this composition.

US Patent No. 3 455 838 describes dry, free-flowing particles containing a water-insoluble substance encapsulated therein, wherein the particles consist essentially of a solid matrix of an encapsulating agent consisting essentially of a dextrinized starch acid-ester of a substituted dicarboxylic acid. A method for encapsulating water-insoluble substances is also described, which comprises making an aqueous dispersion of the encapsulating agent, emulsifying the water-insoluble substance in said dispersion and drying the resulting dispersion to form the dry, free-flowing particles. A preferred encapsulating agent is a dextrinized starch acid-ester of a substituted succinic acid, derived from octenyl succinic acid anhydride.

During the well known process for preparing microcapsules containing an active substance embedded in a matrix material based on starch and modified starch, problems arise because of foaming of the colloidal aqueous dispersion of the starch prior to the addition of the active substance to be dispersed before microencapsulation, and to air-entrapment in the final product. This leads to final products (dispersions or microcapsules), which are not as stable and not as dense as one may wish both for handling, storage and application reasons.

The object of the present invention is to provide an improved dispersion comprising an active substance dispersed in a modified starch as well as an improved process of preparing it. The dispersion is suitable for preparing microcapsules comprising an active substance embedded in a matrix of modified starch.

### SUMMARY OF THE INVENTION

The present invention relates accordingly to a novel dispersion comprising particles of at least one active substance dispersed in an aqueous solution of a modified starch, wherein said dispersion has a pH value in the range 7.5 to 10.

The invention further relates to a process of preparing a dispersion of the invention, which process comprises the steps of
a) providing an aqueous solution of said modified starch,
b) adding to said solution at least one active substance,
c) treating the mixture thus obtained to prepare a dispersion of said at least one active substance in said aqueous solution comprising said modified starch, wherein the process further comprises a step
d) of adjusting pH of said aqueous solution of said modified starch in the range 7.5 to 10 before or after adding said at least one active substance.

The term "dispersion" as used herein covers both an emulsion meaning a mixture comprising liquid particles (oil droplets) dispersed in an aqueous medium and a suspension meaning solid particles dispersed in an aqueous medium, e.g. a finely milled carotenoid or mixtures thereof.

By modified starch to be used according to the invention is meant a starch derived from a natural source, such as potato, wheat, maize, tapioca and rice. Modified starch is prepared from the unmodified starch by (partial) degradation, depolymerisation, hydrolysis, etc. A very appropriate modified starch to be used according to the invention is n-OSA modified starch, e.g. sodium octenyl succinate modified starch, i.e. a starch wherein a part of the hydroxyl groups are esterified by octenyl succinyl acid, providing a surface active property of the starch.

The dispersion of the invention is advantageous due to a reduced tendency of foaming in the dispersion made up by the aqueous solution of modified starch. The resulting dispersion will accordingly be essentially and substantially free of air. The optimal pH depends on the type of modified starch.

The process of the invention for preparation of the dispersion presents production advantages due to the reduced tendency of foaming in the dispersion. In the traditional process, the pH in the dispersion is much lower than neutral pH, normally about 3 to 5, depending of the type of modified starch. It appears that when pH is adjusted in accordance with the invention to about 7.5 to 10, e.g. 7.5 to 9 or 9.5, the foam disappears much faster than during the traditional process.

By the term "essentially" or "substantially" free of air is meant that the amount of included air is so small that the dispersion will boil under vacuum without causing any significant foaming or increase in volume.

The dispersion of the invention possesses a number of advantages, which make it suitable for the production of e.g. microcapsules with improved mechanical and chemical stability and also mechanical strength.

Accordingly, the invention relates in other important aspects to the use of a dispersion for preparing microcapsules as well as a process of preparing microcapsules comprising particles of at least one active substance embedded in a matrix of a modified starch, which process comprises drying of a dispersion of the invention to remove water.

The term "microcapsules" as used herein means particles each comprising a matrix material having embedded therein one single or a plurality of solid or liquid micro particles.

The dispersion of the invention as well as microcapsules prepared from the dispersion has an improved content of solid matter.

It should be emphasised that the pH adjustment suggested according to the present invention is not evident.

The process advantages as well as the improved quality of the final products, provide a more economic production method, due to increased total solid content when the dispersion is dried. Furthermore, it was quite surprising that the pH adjustment had this effect on foaming/air entrapment.

The improved properties of the products comprising the dispersion of the invention will appear from the drawings in which
Figure 1 shows a SEM (Scanning Electron Microscopy) photo of a microcapsule prepared by the traditional process, wherein pH is 4.2, i.e. not adjusted in accordance with the range of the present invention.
Figure 2 shows a SEM photo of a microcapsule prepared from a dispersion of the invention, wherein pH is adjusted to 7.5 before addition of the active substance(s), and
Figure 3 shows a SEM photo of a microcapsule prepared from a dispersion of the invention, wherein pH is adjusted to 9.5 before addition of the active substance(s).

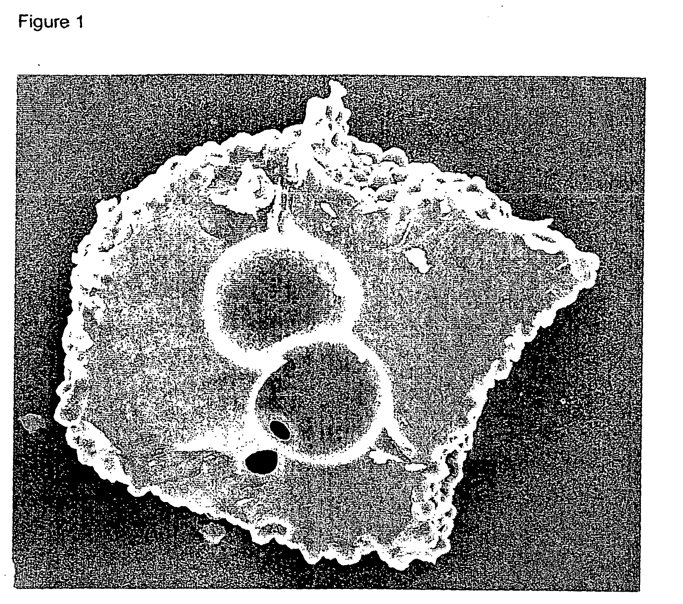

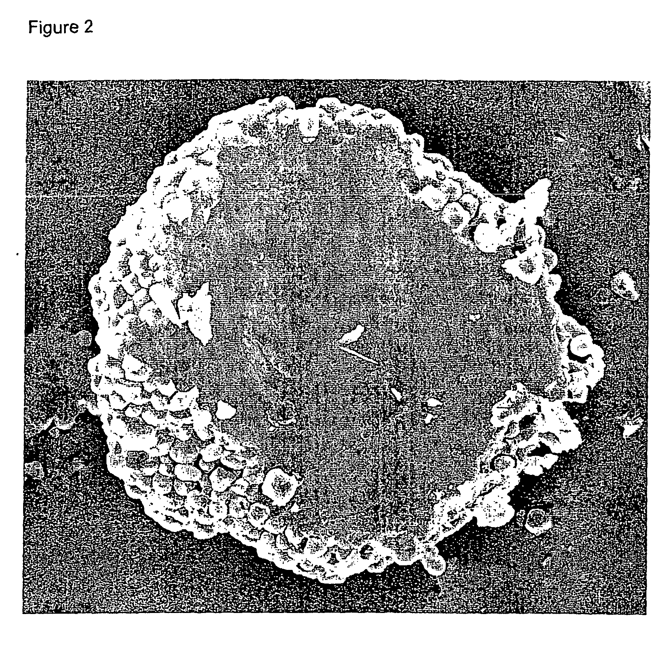

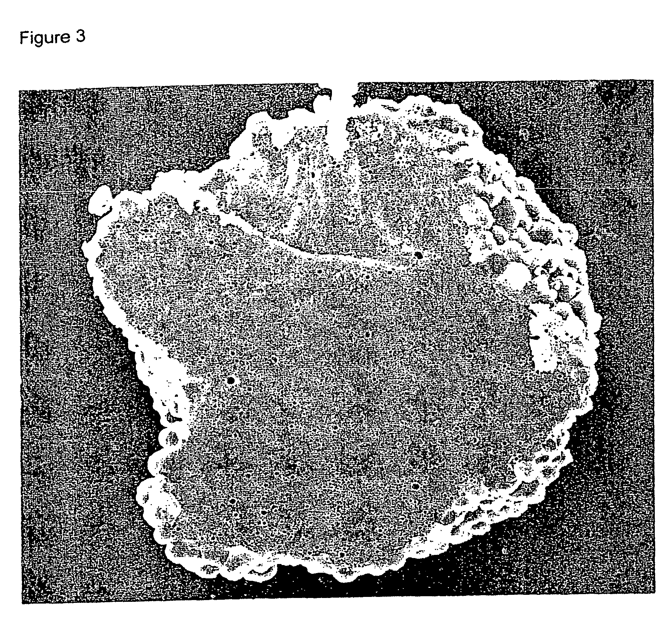

It appears from said photos that microcapsules prepared from a dispersion of the invention are substantially free of air (figures 2 and 3), while the product prepared from a dispersion without pH adjustment contains entrapped air (figure 1).

The photos also show that the matrix material surrounds the encapsulated active substance with a high density thus reducing the degree of oxidation during storage and enhancing the storage stability. Accordingly, the addition of traditional antioxidants can be avoided or reduced, or alternatively, less efficient antioxidant systems may be applied.

### DETAILED DESCRIPTION OF THE INVENTION

In one preferred embodiment of the dispersion of the invention the modified starch is an n-octenyl succinyl acid modified starch, such as sodium octenyl succinate, in which case the pH value is preferable 7.5 to 9.

In another embodiment the dispersion further comprises an antioxidant.

In one embodiment of the invention it relates to a process for preparing microcapsules comprising at least one active substance embedded in a matrix material of a modified starch, which process comprises the steps of
a) providing an aqueous solution of said modified starch,
b) adding to said solution said at least one active substance,
c) treating the mixture thus obtained to prepare a dispersion of particles of said at least one active substance in said aqueous solution comprising said modified starch,
c1) finely dividing and drying the dispersion obtained in step c) to obtain a mass of particles each containing one single or a plurality of liquid or solid micro particles of said at least one active substance embedded in a matrix comprising said modified starch,
   wherein the process further comprises a step
d) of adjusting pH of the aqueous medium of modified starch to the range 7.5 to 10 before or after adding said at least one active substance.

In another preferred embodiment the process comprises a further step e) of removing entrapped air and/or oxygen from the aqueous medium. This removal of entrapped air may for example take place after the adjustment of pH. The removal of air/oxygen may be carried out by any convenient method, such as by evacuation/depressurization. It may also be carried out by steam injection followed by flash cooling (evaporation under vacuum).

In general the adjustment of pH of the aqueous medium in step d) can be affected by an adjustment either before the addition of modified starch to the medium or after the addition of the modified starch and before or after addition of the active substance(s); if necessary it may be adjusted afterwards and currently during the process.

The treatment in step c) of the process of the invention may be carried out by any convenient method, such as homogenising, emulsifying, milling or dispersing.

Step c1) of the process of the invention may be carried out with conventional methods such as spray cooling, spray drying, modified spray drying or sheet drying and crushing, see for example WO 91/06292.

The adjustment of pH in step d) may be carried out by the addition of any suitable alkaline, such as sodium hydroxide.

In one embodiment of the invention pH of the dispersion is readjusted to acid pH in a later step f). In this case the term "later" means that the pH is adjusted in a step taking place later in the process than the pH adjusting in step d). This embodiment, including a readjustment of pH, may be preferred because the resulting dispersion is more stable and have a longer shelf-life at acidic pH, for example in relation to microbiological stability, and because the emulsifying properties of the modified starches are better at low pH.

The applied n-OSA starch could for instance be Capsul® from National Starch and Chemical Company, or any similar product.

The active substances comprised in dispersions of the invention or microcapsules prepared according to the present invention may be any substance, which during storage, transport, handling and use requires protection, e.g. from oxygen, moisture, light radiation, and physical influences, in order to avoid physical and chemical decomposition of the substance. These active substances are further defined as being active in either a chemical or biological system. A protective matrix may be used to prevent the active substance from reacting with other substances present in a composition or with substances with which it may come into contact during use and which would have a deleterious affect upon the active substance's desired property. Also, a protective matrix may be used to transform liquids and other substances, which are difficult to handle, e.g. due to stickiness, into a solid form suitable for handling and processing during use, such as a powder of microcapsules.

Examples of active substances suitable for use in connection with the present invention are fat-soluble substances, such as vitamins, e.g. vitamin A and esters thereof, E and esters thereof, e.g. E-acetate, D and K, fatty acids (both of natural origin and obtained through a fermentative process), e.g. mono- and polyunsaturated fatty acids, which may be added in the form of fish oil containing i.a. the (n-3) fatty acids docosahexaenoic acid (DHA) and eicosapentaenoic acid (EPA), conjugated linolenic acid (CLA), phospholipids derived from e.g. egg yolk and in the form of evening primrose oil and castor oil containing i.a. the (n-6) fatty acid γ-linolenic acid, arachidonic acid, lipoic acid, carotenoids, e.g. β-carotene, lutein, lycopene, β-cryptoxanthin, astaxanthin, Cantaxanthin, citranaxanthin and zeaxanthin, curcumin, benzoquinones, e.g. Coenzyme Q10 (ubidecarenone), phytosterols, oils and fats; water-soluble substances, such as vitamin C; enzymes, e.g. amylase; pharmaceuticals, such as griseofulvin, ibuprofen, benzodiazepines, phenacetin, hormones and paracetamol; and other nutritional supplements, such as minerals and isoflavones. Preferred substances are vitamin A and E and esters thereof, D and K, PUFA oils, carotenoids and Coenzyme Q10. Especially preferred substances are vitamin A and E and esters thereof and D, β-carotene and Coenzyme Q10.

Additional active substances are aroma and flavour compounds.

The non-water soluble material or the matrix material (as the case may be and dependent on the type of additives) may contain conventional additives such as antioxidants, e.g. t-butylhydroxytoluene (BHT), t-butylhydroxyanisole (BHA), ascorbic acid, ascorbyl palmitate, sodium ascorbate, citric acid, sodium citrate, EDTA or its salts, tocopherols, TBHQ, ethoxyquine, propyl gallate, and extracts from herbs, i.a. rosemary or oregano extract; powdering agents, e.g. starches, modified starches, tri-calcium phosphate, lactose, mannitol, ethylcellulose, coagulated albumin, hardened gelatin, casein, stearate-Ca, stearate-Na, metal soaps, hydrogenated ricinus oil, polyoxide, talcum, waxes, and silicates; anti-caking agents, e.g. tri-calcium phosphate and silicates, i.a. silicon dioxide and sodium aluminium silicate; plasticizers, e.g. carbohydrates and carbohydrate alcohols, examples of which are saccharose, glucose, fructose, lactose, invert sugar, sorbitol, mannitol, Trehalose, Tagatose, Pullulan, Raftilose (oligofructose), dextrin, maltodextrin, glycerin, and mixtures thereof, preferably saccharose, Trehalose, Pullulan, dextrin and Raftilose and mixtures thereof.

The invention also relates to microcapsules comprising at least one active substance embedded in a matrix material of a modified starch, which are obtainable by a process of the invention.

The products prepared according to the invention are suitable for a wide variety of applications, such as food, food supplements, beverages, pharmaceutical and veterinary products, feed, feed supplements, personal care products and households products. Accordingly the invention relates to products comprising a dispersion or microcapsule(s) of the invention.

The process of the invention may be carried out in accordance with the following general recipe:

The water soluble ingredients, including the starch material, are added to hot (e.g. 45-70 °C, such as 60-65 °C) ion-exchanged water and dissolved under continuous agitation. The oil soluble ingredients, if any, are added to the heated active substance (e.g. vitamin A-acetate) oil under agitation. The oil phase is then added to the water phase and the mixture is emulsified/homogenised. It may be diluted, if need be, to an appropriate viscosity before it is atomized as indicated above.

The invention will now be described in further detail with reference to the following examples.

### EXAMPLES

### Preparations of dispersion and microcapsules

### Example 1 (pH=7.5)

400 g modified starch (an n-OSA starch from National Starch) and 430 g Isomalt were dissolved in 650 ml heated ion exchanged water at 65°C under continuous agitation. The pH of the solution was adjusted to pH = 7.5 by adding a suitable amount (15 ml) of 4M NaOH. The deflation of air from the foam started at a pH around 5.5.

The solution was evacuated until boiling.

278 g vitamin A-acetate and 12.5 g dl-α-tocopherol was heated to 65°C and mixed well. The oily mixture was added to the aqueous solution and stirred vigorously under nitrogen.

The dispersion was homogenised well and diluted to a sprayable viscosity.

Subsequently the dispersion was atomised in a spray drying tower, where the dispersion particles were covered with a thin layer of starch and dried.

The resulting dry powder had a vitamin A-acetate potency of about 544.000 IU/g.

In order to investigate the stability of the product approximately 3 gram of the product was weighed out, put in a small alu-foil bag and sealed and stored at 25°C/60% RH and 40°C/75% RH for 6 months.

Samples were analysed after 0, 3 and 6 months respectively 0, 1, 2, 3 and 6 months. The results are given below:

| Potency: | 25°C/25%RH | 40°C/75%RH |
|---|---|---|
| Start: | 100% | 100% |
| 1 month: | - | 92 |
| 2 months: | - | 86 |
| 3 months: | 96 | 82 |
| 6 months: | 90 | 69 |

### Example 2 (pH = 9.5):

400 g modified starch (an n-OSA starch from National Starch) and 430 g Isomalt were dissolved in 650 ml ion exchanged water at 65°C under continuous agitation. The pH of the solution was adjusted to pH = 9.5 by adding a suitable amount of (19 ml) 4M NaOH. When pH exceeded approximately 8 the air/foam instantly disappeared completely from the solution.

The solution was evacuated until boiling.

278 g vitamin A-acetate and 12.5 g dl-α-tocopherol was heated to 65°C and mixed well under nitrogen. The oily mixture was added to the aqueous solution and stirred vigorously.

The dispersion was homogenised well and diluted to a sprayable viscosity.

Subsequently the dispersion was atomised in a spray drying tower, where the dispersion particles were covered with a thin layer of starch and dried.

The resulting dry powder had a vitamin A-acetate potency of about 573.000 IU/g.

In order to investigate the stability of the product approximately 3 gram of the product was weighed out, put in a small alu-foil bag and sealed and stored at 25°C/60% RH and 40°C/75% RH for 6 months.

Samples were analysed after 0, 3 and 6 months respectively 0, 1, 2, 3 and 6 months. The results are given below:

| Potency: | 25°C/25%RH | 40°C/75%RH |
|---|---|---|
| Start: | 100% | 100% |
| 1 month: | - | 98 |
| 2 months: | - | 94 |
| 3 months: | 100 | 90 |
| 6 months: | 94 | 81 |

### Comparative example (no adjustment of pH, pH 4.2)

400 g modified starch and 430 g Isomalt were dissolved in 750 ml ion exchanged water at 65°C under continuous agitation. pH of the solution was 4.2. The solution was evacuated until boiling.

278 g vitamin A-acetate and 12.5 g dl-α-tocopherol was heated to 65°C and mixed well. The oily mixture was added to the aqueous solution and stirred vigorously.

The dispersion was homogenised well and diluted to a sprayable viscosity.

Subsequently the dispersion was atomised in a spray drying tower, where the dispersion particles were covered with a thin layer of starch and dried.

The resulting dry powder had a vitamin A-acetate potency of about 563.000 IU/g.

In order to investigate the stability of the product approximately 3 gram of the product was weighed out, put in a small alu-foil bag and sealed and stored at 25°C/60% RH and 40°C/75% RH for 6 months.

Samples were analysed after 0, 3 and 6 months respectively 0, 1, 2, 3 and 6 months. The results are given below:

| Potency: | 25°C/25%RH | 40°C/75%RH |
|---|---|---|
| Start: | 100% | 100% |
| 1 month: | - | 89 |
| 2 months: | - | 82 |
| 3 months: | 95 | 76 |
| 6 months: | 89 | 62 |

### Test of product properties

The pH effect on the properties of the final products was verified by a number of tests conducted on products according to the invention and according to the prior art. During these tests the amount of solid matter was determined. Furthermore the products were subjected to a crushing test, which is a visual method.

In addition, the pH effect was visualised by scanning electron microscopy (SEM).

The results are shown in Table 1 below and in the drawings.

**TABLE 1: pH effect on final products**

| **Emulsion:** | | Example 1 | Example 2 | Comparative Example |
|---|---|---|---|---|
| pH in aqueous medium | | 7.5 | 9.5 | 4.2 |
| Density | g/ml | 1.16 | 1.18 | 1.14 |
| **Powder:** | | | | |
| Crushing test | | Good | Good | Crunchy |
| Density, non-tapped/tapped x100 | g/ml | 0.63/0.76 | 0.66/0.77 | 0.62/0.73 |

### A crushing test is performed as follows:

The product is pressed with a given force between two glass plates and visually judged. The fewer broken particles the stronger is the product.

The SEM photos in Figures 1-3 show that the microcapsules prepared according to examples 1 and 2 are substantially free of air (figures 2 and 3), while the product prepared according to the comparative example contains entrapped air (figure 1).

## Claims

1. A dispersion comprising particles of at least one active substance dispersed in an aqueous solution of a modified starch, wherein said dispersion has a pH value in the range 7.5 to 10.

2. A dispersion according to claim 1, wherein said modified starch is derived from a natural source, such as potato, wheat, maize, tapioca or rice.

3. A dispersion according to any of the claims 1 or 2, wherein said modified starch is chemically or enzymatically modified.

4. A dispersion according to any of the claims 1 to 3, wherein said modified starch is an n-octenyl succinyl acid modified starch.

5. A dispersion according to any of the claims 1 to 4, wherein said dispersion has a pH value in the range 7.5 to 9.5.

6. A dispersion according to any of the claims 1 to 5 further comprising an antioxidant.

7. A process of preparing a dispersion comprising particles of at least one active substance dispersed in an aqueous solution of a modified starch, wherein said dispersion has a pH value in the range 7.5 to 10, which process comprises the steps of
a) providing an aqueous solution of said modified starch,
b) adding to said solution said at least one active substance,
c) treating the mixture thus obtained to prepare a dispersion of particles of said at least one active substance in said aqueous solution comprising said modified starch,
**characterised in that** the process further comprises a step
d) of adjusting pH of the aqueous solution of said modified starch to the range 7.5-10 before or after adding said at least one active substance.

8. A process according to claim 7 for preparing microcapsules comprising at least one active substance embedded in a matrix of a modified starch, which process further comprises the step of
c1) finely dividing and drying the dispersion obtained in step c) to obtain a mass of particles each containing one single or a plurality of liquid or solid micro particles of said at least one active substance embedded in a matrix comprising said modified starch.

9. A process according to any of the claims 7 or 8, wherein said modified starch is derived from a natural source, such as potato, wheat, maize, tapioca or rice.

10. A process according to any of the claims 7 to 9, wherein said modified starch is chemically or enzymatically modified.

11. A process according to any of the claims 7 to 10, wherein said modified starch is an n-octenyl succinyl acid modified starch.

12. A process according to any of the claims 7 to 11, wherein in step d) pH is adjusted to about 7.5 to 9.5.

13. A process according to any of the claims 7 to 12, comprising a further step e) of removing entrapped air and/or oxygen from the aqueous medium.

14. A process according to claim 13, wherein the entrapped air and/or oxygen in the aqueous medium is removed by evacuation/depressurization.

15. A process according to claim 13, wherein the entrapped air in the aqueous medium is removed by steam injection followed by evaporation under vacuum (flash cooling).

16. A process according to any of the claims 7 to 15, wherein the treatment in step c) is effected by homogenising, emulsifying, milling or dispersing.

17. A process according to any of the claims 7 to 16, further comprising the adding of an antioxidant to the aqueous solution.

18. A process according to any of the claims 7 to 17 further comprising the adding of an antioxidant to the non-aqueous solution.

19. A process according to any of the claims 7 to 18, further comprising a step f) of later readjusting pH to acidic conditions, e.g. to 3.5 to 5.

20. A process of preparing microcapsules comprising particles of at least one active substance embedded in a matrix of a modified starch which process comprises drying of a dispersion according to any of the claims 1 to 6 to remove water.

21. Use of a dispersion according to any of the claims 1 to 6 for preparing microcapsules.

22. Microcapsule comprising at least one active substance embedded in a matrix material of a modified starch, **characterised in that** it is obtainable by the process of any of the claims 8 to 20.

23. A product comprising a dispersion according to any of the claims 1 to 6.

24. A product comprising a dispersion obtainable according to any of the claims 7 or 9 to 19.

25. A product comprising microcapsules according to claim 22.

26. A product according to any of the claims 23 to 25 **characterised in that** it is a food, a food supplement, a beverage, a pharmaceutical or veterinary product, a feed or feed supplement, a personal care product or a household product.

## Patentansprüche

1. Dispersion, umfassend Teilchen von wenigstens einer Aktivsubstanz, dispergiert in einer wässrigen Lösung von einer modifizierten Stärke, wobei die Dispersion einen pH-Wert im Bereich 7,5 bis 10 aufweist.

2. Dispersion nach Anspruch 1, wobei die modifizierte Stärke von einem natürlichen Ausgangsstoff wie Kartoffel, Weizen, Mais, Tapioka oder Reis hergeleitet ist.

3. Dispersion nach einem der Ansprüche 1 oder 2, wobei die modifizierte Stärke chemisch oder enzymatisch modifiziert ist.

4. Dispersion nach einem der Ansprüche 1 bis 3, wobei die modifizierte Stärke eine n-Octenyl-succinylsäure-modifizierte Stärke ist.

5. Dispersion nach einem der Ansprüche 1 bis 4, wobei die Dispersion einen pH-Wert im Bereich von 7,5 bis 9,5 aufweist.

6. Dispersion nach einem der Ansprüche 1 bis 5, außerdem umfassend ein Antioxidationsmittel.

7. Verfahren zum Herstellen einer Dispersion, umfassend Teilchen von wenigstens einer Aktivsubstanz, dispergiert in einer wässrigen Lösung von einer modifizierten Stärke, wobei die Dispersion einen pH-Wert im Bereich von 7,5 bis 10 aufweist, wobei das Verfahren die Schritte umfasst:
a) Bereitstellen einer wässrigen Lösung der modifizierten Stärke,
b) Zugeben der wenigstens einen Aktivsubstanz zu der Lösung,
c) Behandeln der so erhaltenen Mischung, um eine Dispersion von Teilchen der wenigstens einen Aktivsubstanz in der wässrigen Lösung, die die modifizierte Stärke umfasst, herzustellen,
**dadurch gekennzeichnet, dass** das Verfahren außerdem einen Schritt umfasst:
d) Einstellen des pH der wässrigen Lösung der modifizierten Stärke auf den Bereich 7,5 bis 10 vor oder nach dem Zugeben der wenigstens einen Aktivsubstanz.

8. Verfahren nach Anspruch 7 zum Herstellen von Mikrokapseln, umfassend wenigstens eine Aktivsubstanz, eingebettet in eine Matrix aus einer modifizierten Stärke, wobei das Verfahren außerdem den Schritt umfasst:
c1) Feinverteilen und Trocknen der in Schritt c) erhaltenen Dispersion, um eine Masse von Teilchen zu erhalten, die jeweils ein einzelnes oder eine Vielzahl von flüssigen oder festen Mikroteilchen der wenigstens einen Aktivsubstanz, eingebettet in eine Matrix, die die modifizierte Stärke umfasst, enthalten.

9. Verfahren nach einem der Ansprüche 7 oder 8, wobei die modifizierte Stärke von einem natürlichen Ausgangsstoff, wie Kartoffel, Weizen, Mais, Tapioka oder Reis hergeleitet ist.

10. Verfahren nach einem der Ansprüche 7 bis 9, wobei die modifizierte Stärke chemisch oder enzymatisch modifiziert ist.

11. Verfahren nach einem der Ansprüche 7 bis 10, wobei die modifizierte Stärke eine n-Octenyl-succinylsäure-modifizierte Stärke ist.

12. Verfahren nach einem der Ansprüche 7 bis 11, wobei in Schritt d) der pH auf ungefähr 7,5 bis 9,5 eingestellt wird.

13. Verfahren nach einem der Ansprüche 7 bis 12, umfassend einen weiteren Schritt:
e) Entfernen von eingeschlossener Luft und/oder Sauerstoff aus dem wässrigen Medium.

14. Verfahren nach Anspruch 13, wobei die eingeschlossene Luft und/oder der Sauerstoff in dem wässrigen Medium durch Evakuierung/Druckabbau entfernt wird.

15. Verfahren nach Anspruch 13, wobei die eingeschlossene Luft in dem wässrigen Medium durch Dampfinjektion, gefolgt von Verdampfung unter Vakuum (Entspannungskühlung), entfernt wird.

16. Verfahren nach einem der Ansprüche 7 bis 15, wobei die Behandlung in Schritt c) durch Homogenisieren, Emulgieren, Vermahlen oder Dispergieren bewirkt wird.

17. Verfahren nach einem der Ansprüche 7 bis 16, außerdem umfassend das Zugeben eines Antioxidationsmittels zu der wässrigen Lösung.

18. Verfahren nach einem der Ansprüche 7 bis 17, außerdem umfassend das Zugeben eines Antioxidationsmittels zu der nichtwässrigen Lösung.

19. Verfahren nach einem der Ansprüche 7 bis 18, außerdem umfassend einen Schritt:
f) späteres Wiedereinstellen des pH-Werts auf saure Bedingungen, z. B. auf 3,5 bis 5.

20. Verfahren zum Herstellen von Mikrokapseln, umfassend Teilchen von wenigstens einer Aktivsubstanz, eingebettet in eine Matrix aus einer modifizierten Stärke, wobei das Verfahren das Trocknen einer Dispersion nach einem der Ansprüche 1 bis 6 zum Entfernen von Wasser umfasst.

21. Verwendung einer Dispersion nach einem der Ansprüche 1 bis 6 zum Herstellen von Mikrokapseln.

22. Mikrokapsel, umfassend wenigstens eine Aktivsubstanz, eingebettet in ein Matrixmaterial aus einer modifizierten Stärke, **dadurch gekennzeichnet, dass** sie durch das Verfahren nach einem der Ansprüche 8 bis 20 erhältlich ist.

23. Produkt, umfassend eine Dispersion nach einem der Ansprüche 1 bis 6.

24. Produkt, umfassend eine Dispersion, erhältlich nach einem der Ansprüche 7 oder 9 bis 19.

25. Produkt, umfassend Mikrokapseln nach Anspruch 22.

26. Produkt nach einem der Ansprüche 23 bis 25, **dadurch gekennzeichnet, dass** es ein Nahrungsmittel, ein Nahrungsergänzungsmittel, ein Getränk, ein pharmazeutisches oder tierärztliches Produkt, ein Futter oder Futterergänzungsmittel, ein Körperpflegeprodukt oder ein Haushaltsprodukt ist.

## Revendications

1. Dispersion comprenant des particules d'au moins une substance active dispersée dans une solution aqueuse d'un amidon modifié, dans laquelle ladite dispersion a une valeur de pH comprise dans la gamme allant de 7,5 à 10.

2. Dispersion selon la revendication 1, dans laquelle ledit amidon modifié est dérivé d'une source naturelle, telle que la pomme de terre, le blé, le maïs, le tapioca ou le riz.

3. Dispersion selon l'une quelconque des revendications 1 ou 2, dans laquelle ledit amidon modifié est de l'amidon modifié par voie chimique ou enzymatique.

4. Dispersion selon l'une quelconque des revendications 1 à 3, dans laquelle ledit amidon modifié est un amidon modifié au n-octénylsuccinyl-acide.

5. Dispersion selon l'une quelconque des revendications 1 à 4, dans laquelle ladite dispersion a une valeur de pH comprise entre 7,5 et 9,5.

6. Dispersion selon l'une quelconque des revendications 1 à 5, comprenant en outre un antioxydant.

7. Procédé de préparation d'une dispersion comprenant des particules d'au moins une substance active dispersée dans une solution aqueuse d'un amidon modifié, dans lequel ladite dispersion a une valeur de pH dans la gamme allant de 7,5 à 10, ledit procédé comprenant les étapes consistant à
a) fournir une solution aqueuse dudit amidon modifié,
b) ajouter à ladite solution ladite substance active, au moins au nombre de une,
c) traiter le mélange ainsi obtenu pour préparer une dispersion de particules de ladite substance active, au moins au nombre de une, dans ladite solution aqueuse comprenant ledit amidon modifié,
**caractérisé en ce que** ledit processus comprend en outre une étape consistant à
d) ajuster le pH de la solution aqueuse dudit amidon modifié dans la gamme de 7,5 à 10 avant ou après l'ajout de ladite substance active, au moins au nombre de une.

8. Procédé selon la revendication 7 pour préparer des microcapsules comprenant au moins une substance active imbriquée dans une matrice d'un amidon modifié, ledit procédé comprenant en outre l'étape consistant à
c1) diviser finement et sécher la dispersion obtenue à l'étape c) pour obtenir une masse de particules contenant chacune une ou plusieurs microparticules liquides ou solides de ladite substance active, au moins au nombre de une, imbriquée(s) dans une matrice contenant ledit amidon modifié.

9. Procédé selon l'une quelconque des revendications 7 ou 8, dans lequel ledit amidon modifié est dérivé d'une source naturelle, telle que la pomme de terre, le blé, le maïs, le tapioca ou le riz.

10. Procédé selon l'une quelconque des revendications 7 à 9, dans lequel ledit amidon modifié est modifié par voie chimique ou enzymatique.

11. Procédé selon l'une quelconque des revendications 7 à 10, dans lequel ledit amidon modifié est un amidon modifié au n-octénylsuccinyl-acide.

12. Procédé selon l'une quelconque des revendications 7 à 11, dans lequel dans l'étape d), le pH est ajusté entre environ 7,5 et 9,5.

13. Procédé selon l'une quelconque des revendications 7 à 12, comprenant une autre étape e) consistant à retirer du milieu aqueux l'air et/ou l'oxygène piégé.

14. Procédé selon la revendication 13, dans lequel l'air et/ou l'oxygène piégé dans le milieu aqueux est retiré par évacuation/dépressurisation.

15. Procédé selon la revendication 13, dans lequel l'air piégé dans le milieu aqueux est retiré par injection de vapeur suivie d'une évaporation sous vide (refroidissement ultra-rapide).

16. Procédé selon l'une quelconque des revendications 7 à 15, dans lequel le traitement de l'étape c) est effectué par homogénéisation, mise en émulsion, broyage ou dispersion.

17. Procédé selon l'une quelconque des revendications 7 à 16, comprenant en outre l'addition d'un antioxydant à la solution aqueuse.

18. Procédé selon l'une quelconque des revendications 7 à 17, comprenant en outre l'addition d'un antioxydant à la solution non aqueuse.

19. Procédé selon l'une quelconque des revendications 7 à 18, comprenant en outre une étape f) consistant à réajuster ultérieurement le pH à des conditions acides, par exemple entre 3,5 et 5.

20. Procédé de préparation de micro-capsules comprenant des particules d'au moins une substance active imbriquée dans une matrice d'un amidon modifié, ledit procédé comprenant le séchage d'une dispersion selon l'une quelconque des revendications 1 à 6 pour supprimer l'eau.

21. Utilisation d'une dispersion selon l'une quelconque des revendications 1 à 6 pour préparer des micro-capsules.

22. Micro-capsule comprenant au moins une substance active imbriquée dans un matériau de matrice d'un amidon modifié, **caractérisée en ce qu'**elle peut être obtenue par le procédé selon l'une quelconque des revendications 8 à 20.

23. Produit comprenant une dispersion selon l'une quelconque des revendications 1 à 6.

24. Produit comprenant une dispersion pouvant être obtenue selon l'une quelconque des revendications 7 ou 9 à 19.

25. Produit comprenant des micro-capsules selon la revendication 22.

26. Produit selon l'une quelconque des revendications 23 à 25, **caractérisé en ce qu'**il s'agit d'un aliment, d'un complément alimentaire, d'une boisson, d'un produit pharmaceutique ou vétérinaire, d'un aliment ou d'un complément alimentaire pour animaux, d'un produit de soin des personnes ou d'un produit ménager.
